# EUROPÄISCHE PATENTSCHRIFT

(11) **EP 1 838 699 B1**
(45) Veröffentlichungstag und Bekanntmachung des Hinweises auf die Patenterteilung: **28.04.2010**
(21) Anmeldenummer: 05850162.8
(22) Anmeldetag: 15.12.2005
(51) Int. Cl.: C07D 403/10

(54) **VERFAHREN ZUR HERSTELLUNG VON CANDESARTAN**
METHOD FOR PRODUCTION OF CANDESARTAN
PROCEDE DE PRODUCTION DE CANDESARTAN

(30) Priorität: 16.12.2004 DE 102004060699
(43) Veröffentlichungstag der Anmeldung: 03.10.2007
(73) Patentinhaber: Ratiopharm GmbH, 89079 Ulm (DE)
(72) Erfinder: WANG, Yaping, Lanzhou City Gansu Province 730020 (CN); LI, Yonggang, Pudong district, Shangai 201203 (CN); LI, Yulin, Lanzhou City Gansu Province 730000 (CN); ZHENG, Guojun, Dai Shan County Zhejiang Province 316200 (CN); LI, Yi, Zheng Ning County Gansu Province 745300 (CN); KÖCHER, Stefan, 89077 Ulm (DE)
(74) Vertreter: Breuer, Markus
(86) Internationale Anmeldenummer: PCT/DE2005/002267
(87) Internationale Veröffentlichungsnummer: WO 2006/063578

(56) Entgegenhaltungen:
- WO-A-93/10106
- WO-A-2004/065383
- CN-A- 1 510 031
- LARSEN R D ET AL: "Efficient Synthesis of Losartan, A Nonpeptide Angiotensin II Receptor Antagonist" JOURNAL OF ORGANIC CHEMISTRY, AMERICAN CHEMICAL SOCIETY. EASTON, US, Bd. 59, Nr. 21, 21. Oktober 1994 (1994-10-21), Seiten 6391-6394, XP002097895 ISSN: 0022-3263

## Beschreibung

Die vorliegende Erfindung betrifft neue Verfahren zur Herstellung von Candesartan oder einer geschützten Form von Candesartan, eines Candesartan-Salzes oder eines Candesartan-Esters

Der Wirkstoff Candesartan ist ein Angiotensin-II-Antagonist, der den Angiotensin-II-Rezeptor vom Typ 1 hemmt und für die Behandlung der essentiellen Hypertonie zugelassen wurde. Candesartan zeigt eine gute Verträglichkeit und kann in Form von Candesartan Cilexetil peroral appliziert werden.

Die Verbindung Candesartan (chemischer Name 2-Ethoxy-1-[[2'-(1*H*-tetrazol-5-yl)biphenyl-4-yl]-methyl]-1*H*-benzimidazol-7-carbonsäure) und deren Synthese wurden erstmals in EP 0 459 136 beschrieben. Candesartan wird üblicherweise nicht als freie Carbonsäure, sondern als der 1-{[(Cyclohexyloxy)carbonyl]oxy}-ethylester vertrieben, auch Candesartan Cilexetil genannt. Gemäß EP 0 459 136 wird bei der Herstellung von Candesartan von bereits vorgebildeten Biphenylderivaten ausgegangen.

Auch nach der Lehre von EP 0 881 212 wird bei der Synthese von Candesartan von bereits vorgebildeten Biphenylderivaten ausgegangen.

CN 1 510 031 A beschreibt eine C-GKupplung, bei der 2-Ethoxy-1-(p-halophenyl)-methyl-1H-benzimidazol-7-carbonsäure-1-[[(Cyclohexyloxy)carbonyl]oxy]ethylester mit 5-(2-Halophenyl)-2-(1H)-tetrazol mittels Grignardreaktion zu Candesartan Cilexetil umgesetzt wird. Dabei kommt ein Nickelkatalysator, Cl₂Ni(PPh₃)₂ zum Einsatz.

WO 2004/065383 betrifft ein Verfahren zur Herstellung von Irbesartan. Dazu werden spezielle Zwischenprodukte und Reaktionsbedingungen offenbart.

WO 93/10106 betrifft die Herstellung bestimmter Verbindungen, darunter auch Verbindungen mit einer Biphenylstruktur. In dem Dokument findet sich allerdings kein Hinweis auf Candesartan.

Larsen et al., J. Org. Chem., 59 (1994) 6391-6394 betrifft eine Synthese für Losartan. Losartan unterscheidet sich strukturell von Candesartan, und die Entgegenhaltung enthält keinen Hinweis, dass die dort beschriebene Synthese für Candesartan anwendbar sein könnte.

Aufgabe der Erfindung ist es, neue Verfahren zur Bereitstellung von Candesartan in Form der freien Carbonsäure, eines Salzes, eines Esters oder einer geschützten Form von Candesartan zur Verfügung zu stellen. Dabei soll nach Möglichkeit die Verwendung giftiger, allergener, karzinogener und/oder teratogener Verbindungen verzichtet werden.

Die vorstehende Aufgabe wird gelöst durch ein Verfahren zur Herstellung von Candesartan, einer geschützten Form von Candesartan, eines Candesartan-Salzes oder eines Candesartan-Esters, insbesondere Candesartan Cilexetil, das folgende Schritte umfasst:
(a) Bereitstellen und Umsetzen einer Verbindung der Formel (I) 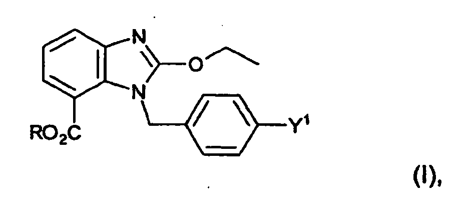 worin
   - R Wasserstoff, ein unsubstituierter oder substituierter Alkyl- oder Arylrest, (Cyclohexyloxycarbonyloxy)ethyl, vorzugsweise Methyl ist,
   - Y¹ eine Gruppe ist, die unter Bildung einer C-C-Bindung in eine Kupplungsreaktion einzugehen vermag, in die ferner eine Gruppe Y² eingeht,
      mit einer die Gruppe Y² aufweisenden Verbindung der Formel (II) 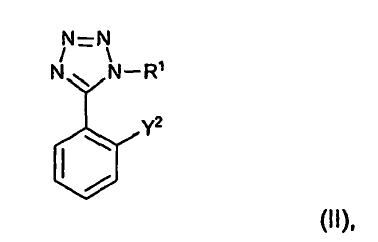
   worin R¹ eine Tetrazolylschutzgruppe oder Wasserstoff ist,
   unter Bildung einer geschützten Form von Candesartan oder Candesartan Cilexetil oder eines anderen Candesartan-Esters, wobei Y¹ und Y² wie in Anspruch 1 definiert sind und gegebenenfalls
(b) Umwandeln in Candesartan, Candesartan Cilexetil oder in ein physiologisch verträgliches Salz.

Vorteilhaft ist ein erfindungsgemäßes Verfahren, in dem in Formel (I) der Rest R ein C₁ bis C₄-Alkylrest, insbesondere ein Methylrest ist.

Wenn in Formel (I) R ein C₁ bis C₄-Alkylrest, insbesondere ein Methylrest,ist, dann liegt eine gegenüber einer Verbindung mit R = (Cyclohexyloxycarbonyloxy)ethyl vergleichsweise gering funktionalisierte Verbindung vor, die unempfindlicher gegenüber den jeweiligen Reaktionsbedingungen ist. Wegen der dann vergleichsweise geringen Funktionalisierung ist das Edukt auch weniger dazu geeignet, die Aktivität der bei der Reaktion verwendeten Reagenzien und/oder Katalysatoren zu mindern. Eine solche Beeinträchtigung könnte beispielsweise durch Komplexierung von bei der Reaktion eingesetzten Metallen oder Metall enthaltenden Verbindungen durch die freien Elektronenpaare der Sauerstoffatome eines (Cyclohexyloxycarbonyloxy)ethylrestes eintreten. Auf diese Weise lässt sich die Zahl bzw. Menge von Nebenprodukten verringern und die Ausbeute erhöhen.

Falls in Formel (I) R nicht Wasserstoff ist, umfasst Schritt (b) des erfindungsgemäßen Verfahrens die Hydrolyse des aus Schritt (a) resultierenden Esters, vorzugsweise mittels Behandlung mit NaOH in EtOH.

Der Einsatz anderer Mittel zur Esterhydrolyse ist jedoch ebenfalls möglich. Der Fachmann wird solche Mittel auszuwählen wissen.

Schritt (b) kann erfindungsgemäß außerdem das Umsetzen von Candesartan mit einer Verbindung der Formel (IV) 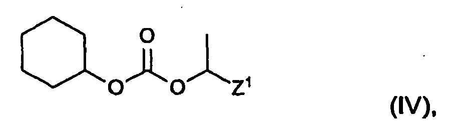 worin Z¹ eine Abgangsgruppe ist, unter Bildung von Candesartan Cilexetil umfassen, vorzugsweise in Anwesenheit von Nal und K₂CO₃.

In erfindungsgemäßen Verfahren kann R¹ ausgewählt sein aus Wasserstoff, *tert-*Butyl und Triphenylmethyl. Bevorzugt ist R¹ Triphenylmethyl.

Falls in Formel (I) R Methyl ist, entsteht Candesartan-Methylester, der durch Umsetzen mit NaOH in EtOH in Candesartan, bzw. ein Candesartan-Salz, überführt werden kann, das wiederum in Candesartan Cilexetil überführbar ist.

In einer bevorzugten Ausführungsform erfolgt die Umsetzung der Verbindung der alligemeinen Formel (I) mit der Verbindung der allgemeinen Formel (II) in einem molaren Verhältnis von 0,2 : 1 bis 2 : 1, besonders bevorzugt von 0,3 : 1 bis 0,8 : 1.

Im erfindungsgemäßen Verfahren führt die Reaktion der Reste Y¹ und Y² zu einer sogenannten C-C Kupplung.

In erfindungsgemäßen Verfahren kann oder können ein oder mehrere Katalysatoren, vorzugsweise umfassend ein oder mehrere Übergangsmetalle, insbesondere Mangan, Chrom, Eisen, Kobalt, Nickel oder Palladium eingesetzt werden. Diese Katalysatoren katalysieren insbesondere die C-C Kupplungsreaktion.

Der Einsatz derartiger Katalysatoren ermöglicht eine besonders wirtschaftliche Durchführung des Verfahrens. Der Katalysator wird üblicherweise in einer Menge von 0,001 mol-% bis 20 mol-%, bevorzugt von 0,01 bis 15 und insbesondere 0,1 bis 10 mol-%, bezogen auf die molare Menge an Verbindung gemäß Formel (I), verwendet.

Der oder die Katalysatoren kann oder können ausgewählt sein aus MnCl₂, CrCl₃, FeCl₂, Fe(acac)₃, FeCl₃, Fe(salen)Cl, CoCl₂(dppe), CoCl₂(dpph), Co(acac)₂, CoCl₂(dppb), Pd(PPh₃)₄, NiCl₂(PPh₃)₂.

Besonders bevorzugt wird Pd(PPh₃)₄ oder NiCl₂(PPh₃)₂.

In einer bevorzugten Ausführungsform können die verwendeten Katalysatoren zusammen mit einem Aktivator eingesetzt werden. Dieser Aktivator überführt die Metallatome der Katalysatoren in die Oxidationsstufe null.

Beispiele für derartige Aktivatoren sind Zink (bevorzugt in Form von Zinkpulver), Natriumborhydrid, Lithiumaluminiumhydrid oder organische Verbindungen von Aluminium, Magnesium oder Lithium (bevorzugt Butyllithium oder DIBAH).

Üblicherweise liegt das Mengenverhältnis von Aktivator zu Katalysator bei 25 : 1 bis 1 : 1, bevorzugt von 18 : 1 bis 2 : 1.

In einer weiteren bevorzugten Ausführungsform können die verwendeten Katalysatoren zusammen mit einem Stabilisator eingesetzt werden. Dieser Stabilisator stabilisiert die Metallatome der Katalysatoren in der Oxidationsstufe null.

Beispiele für derartige Stabilisatoren sind Lewis-Basen, bevorzugt Phosphane, besonders bevorzugt Triarylphosphane und Trialkylphosphane, insbesondere Triphenylphosphan.

Üblicherweise liegt das Mengenverhältnis von Stabilisator zu Katalysator bei 10 : 1 bis 1 : 1, bevorzugt von 5 : 1 bis 1,5 : 1.

Insbesondere ist es bevorzugt, dass Katalysator, Aktivator und Stabilisator gemeinsam eingesetzt werden.

Die Verwendung von derartigen Katalysatoren in C-C-Kupplungsreaktionen, welche Eisen, Mangan, Chrom oder Kobalt enthalten ist von besonderem Vorteil, da die darin enthaltenen Metalle vergleichsweise günstig sind.

In einer alternativen Ausführungsform kann der Katalysator oder die Katalysatoren ausgewählt sein aus der Gruppe der phosphanfreien, vorzugsweise Eisen enthaltenden Katalysatoren. Dadurch werden Nachteile vermieden, die mit dem Einsatz von phosphanhaltigen Katalysatoren einhergehen, nämlich insbesondere deren Giftigkeit, deren Tendenz, sich mit Luftsauerstoff zu verbinden, und die damit einhergehende Gefahr der Selbstentzündung.

In erfindungsgemäßen Verfahren können außerdem eines oder mehrere der folgenden Lösungsmittel eingesetzt werden: THF (Tetrahydrofuran), THF/NMP (N-Methylpyrrolidon), Et₂O (Dietylether), DME (Dimethoxyethan), Benzol und Toluol. Besonders bevorzugt ist THF. Die Lösungsmittel können gegebenenfalls im Gemisch mit Wasser eingesetzt werden.

Ein Verfahren zur Herstellung einer Verbindung mit der oben definierten Formel (I) umfasst folgende Schritte:

Bereitstellen und Umsetzen einer Verbindung der Formel (III) 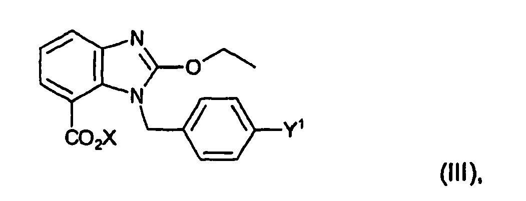 worin
- Y¹ dieselbe Bedeutung hat wie oben;
- X eine Gruppe ist, die unter Bildung einer O-C-Bindung in eine Reaktion einzugehen vermag, in die ferner eine Gruppe Z¹ eingeht,
   mit einer Verbindung der Formel (IV) 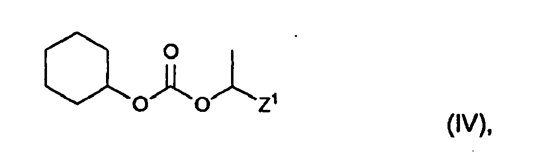
worin Z¹ eine Abgangsgruppe ist,
unter Bildung einer Verbindung der Formel (I).
X kann ein Alkalimetall oder vorzugsweise Wasserstoff sein und/oder Z¹ kann für ein Halogen, vorzugsweise Jod, stehen.

Ein Verfahren zur Herstellung einer Verbindung mit der oben definierten Formel (III) umfasst folgenden Schritt:

Bereitstellen und Entschützen einer Verbindung der Formel (V) 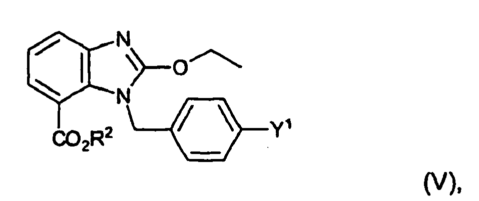 worin
- Y¹ dieselbe Bedeutung hat wie oben, und
- R² eine unter Bildung einer Verbindung der Formel (III) gegen X austauschbare Gruppe ist, wobei X dieselbe Bedeutung hat wie oben im Zusammenhang mit Formel (III) dargelegt.

R² kann aus einer der folgenden funktionellen Gruppen ausgewählt sein: substituiertes oder unsubstituiertes C₁-C₆-Niederalkyl, Benzyl, oder Aryl, vorzugsweise Ethyl (CH₂CH₃), und noch bevorzugter Methyl (CH₃).

Ein Verfahren zur Herstellung einer Verbindung mit der oben definierten Formel (V) umfasst folgenden Schritt:

Bereitstellen und Umsetzen einer Verbindung der Formel (VI) 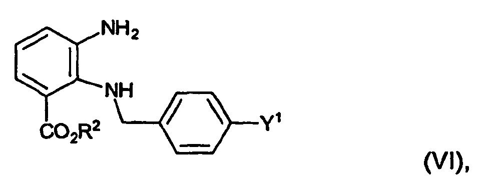 worin Y¹ und R² dieselbe Bedeutung haben wie oben,
mit einem carbonylierenden Reagenz oder vorzugsweise C(OEt)₄, unter Bildung einer Verbindung der Formel (V).

In einem solchen Verfahren kann ferner bei der Umsetzung Ac₂O verwendet werden.

Ein Verfahren zur Herstellung einer Verbindung der Formel (VI) wie oben definiert umfasst folgenden Schritt:

Bereitstellen einer Verbindung der Formel (VII) 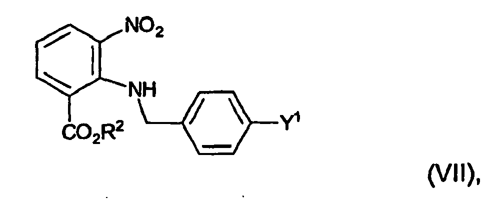 worin Y¹ und R² dieselbe Bedeutung haben wie oben, und

Umwandeln der darin vorhandenen Nitrogruppe in eine Amingruppe.

Das Umwandeln der Nitro- in die Amingruppe kann unter Zuhilfenahme unedler Metalle, katalytischer Hydrierung, auf elektrolytischem Wege oder bevorzugt unter Zuhilfenahme von SnCl₂ bewerkstelligt werden.

Ein Verfahren zur Herstellung einer Verbindung der Formel (VII) wie oben definiert weist folgenden Schritt auf:

Bereitstellen und Entschützen einer Verbindung der Formel (VIII) 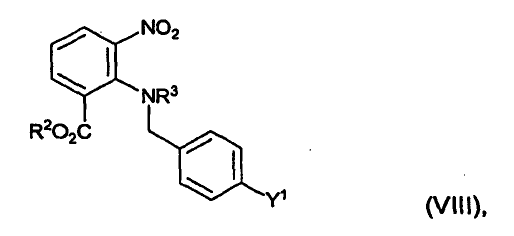 worin Y¹ und R² dieselbe Bedeutung haben wie oben und worin R³ eine gegen H austauschbare Schutzgruppe ist,
unter Bildung einer Verbindung der Formel (VII) wie oben definiert.

R³ kann eine Carboxy-Alkylgruppe, vorzugsweise eine Carboxy-tert-Butylgruppe (-COOC-(CH₃)₃) sein.

Ein Verfahren zur Herstellung einer Verbindung der Formel (VIII) wie oben definiert weist folgenden Schritt auf:

Bereitstellen und Umsetzen einer Verbindung der Formel (IX) 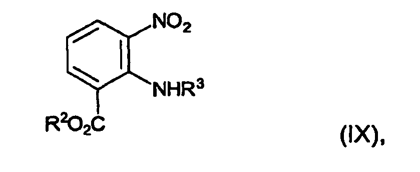 worin R² und R³ dieselbe Bedeutung haben wie oben,
mit einer Verbindung der Formel (X) 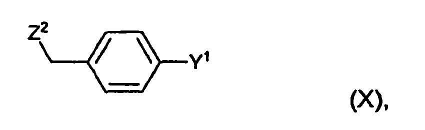 worin
- Y¹ dieselbe Bedeutung hat wie oben, und
- Z² eine Abgangsgruppe ist,
unter Bildung einer Verbindung der Formel (VIII).

Z² kann ausgewählt sein aus einer der folgenden funktionellen Gruppen: CI, I und vorzugsweise Br.

Die Umsetzung einer Verbindung der Formel (IX) mit einer Verbindung der Formel (X) kann in Gegenwart von basischen Verbindungen, bevorzugt Alkali- oder Erdalkalicarbonate, insbesondere Na₂CO₃ oder K₂CO₃ durchgeführt werden.

### Ausführungsbeispiele

Die folgenden Ausführungsbeispiele haben die Darstellung von Verbindungen **(e), (g), (h), (i)** und **(j),** zum Gegenstand. Die jeweiligen Verbindungen der Formeln **(e), (g), (h), (i)** und **(j)** entsprechen jeweils Zwischenprodukten mit den Formeln (VIII), (VI), (V), (I), und (III) mit Y¹ = Br, R² = Methyl, R₃ = CO₂*t*-Bu, X = H, R = {[(Cyclohexyloxy)carbonyl]oxy}ethyl.

Die Zielverbindung Candesartan bzw. Candesartan Cilexetil lässt sich ausgehend von den jeweiligen Zwischenverbindungen darstellen, wie für den Fachmann an Hand der Ausführungsbeispiele unschwer zu erkennen ist.

### Allgemeine Reaktionsbedingungen

Alle trockenen Lösungsmittel (CH₂Cl₂, THF, Et₂O, Benzol, Toluol, DMF, MeCN) wurden nach Standardmethoden getrocknet, d.h. durch Entfernen von Wasser und Sauerstoff und Destillation vor Benutzung. Die im Folgenden beschriebenen Reaktionen wurden, sofern nötig, unter Inertgasatmosphäre (N₂ oder Ar) durchgeführt und mittels Dünnschichtchromatographie (*Thin Layer Chromatography*, TLC) überwacht. Bei den Extraktionen lassen sich Diethylether, Ethylacetat oder Chloroform verwenden. Die Extrakte wurden auf übliche Weise getrocknet, etwa unter Zuhilfenahme von wasserfreiem MgSO₄ oder Na₂SO₄, soweit nicht anders angegeben. Die Reaktionsprodukte wurden, falls nötig, mittels Säulenchromatographie unter Verwendung von beispielsweise Petrolether (60 - 90°C) Ethylacetat oder Petrolether (30 - 60°C) / Ethylacetat als Eluenten gereinigt. Wenn Platten des Typs GF₂₅₄ für die TLC benutzt wurden, wurde als Detektionsmittel lod oder eine ethanolische Lösung von Phosphormolybdänsäure verwendet. Das Silicagel für die Chromatographie (200 - 300 Körnung) und TLC (GF₂₅₄) wurde hergestellt von *Qingdao Sea Chemical Factory* und *Yantai Chemical Factory*. Alle Lösungsmittel und Reagenzien waren von analytischer oder chemischer Reinheit.

Die Schmelzpunktbestimmung erfolgt mittels eines XT₄-100x *micro-melting point tester*. Die Aufnahme von Infrarotspektren erfolgte anhand von KBr-Presslingen oder PE-Filmen auf Nicolet AVATAR 360 FT-IR- und Nicolet NEXUS 670 FT-IR-Spektrometern. NMR-Messungen erfolgten an NMR-Spektrometem der Firmen Varian (Mercury-300) und Bruker (AM-400) mit SiMe₄ als internem Standard in CDCl₃, soweit nicht anders vermerkt. LRMS wurden bestimmt mit einem HP-5988 Massenspektrometer unter Verwendung von EI bei 70eV, soweit nicht anders angegeben. HRMS wurden gemessen mit einem Bruker Daltonics APEX II 47e FT-ICR Massenspektrometer.

### 1. Darstellung von

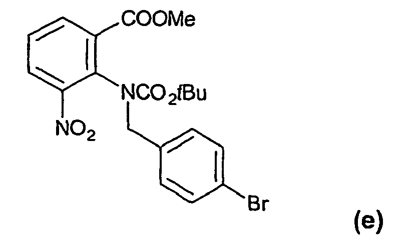

a) 3-Nitrophthalsäure (35 g) wurde in 215 ml Methanol, enthaltend 20 ml konzentrierte H₂SO₄, in einem 500 ml Rundkolben gelöst. Nach 24 h Sieden unter Rückfluss wurde die Reaktionsmischung im Vakuum eingeengt. Mittels einer wässrigen, gesättigten K₂CO₃-Lösung wurde der pH-Wert des Rückstandes auf pH 11 - 12 eingestellt. Anschließend wurde die Reaktionsmischung mit Ethylacetat extrahiert und der pH-Wert der wässrigen Phase wurde mit konzentrierter Salzsäure auf pH 2 - 3 eingestellt. Der Rückstand wurde extrahiert (2 x 1.000 ml CH₂Cl₂). Die organischen Phasen wurden vereinigt und mit Wasser und einer wässrigen NaCI-Lösung gewaschen, über MgSO₄ getrocknet und eingeengt. Der erhaltene gelbe Feststoff kann ohne weitere Reinigung direkt weiter verwendet werden; das Produkt hat die Formel 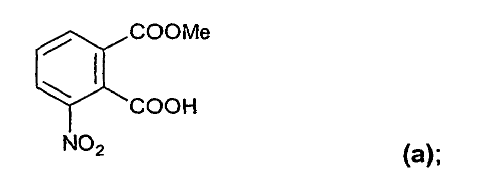
b) 30 g Verbindung **(a)** aus Schritt a) und 16 ml SOCl₂ wurden in 200 ml trockenem Benzol in einem 500 ml Rundkolben gelöst. Die Mischung wurde 3h unter Rückfluss gekocht und anschließend eingeengt, wobei ein weißes Pulver entstand; das Produkt hat die Formel 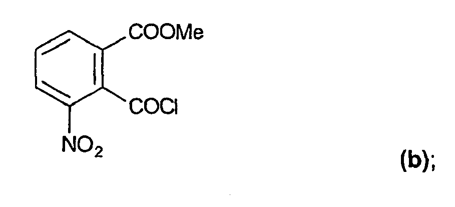
c) 30 g Verbindung **(b)** aus Schritt b) wurden in 120 ml Aceton in einem 500 ml Rundkolben gelöst. Eine wässrige Lösung von 120 ml NaN₃ (184 mmol, 12 g) wurde der Reaktionsmischung langsam zugetropft. Anschließend wurde die Reaktionsmischung eine Stunde gerührt. Die Mischung wurde filtriert, mit Eiswasser gewaschen und im Vakuum getrocknet. Das erhaltene Produkt hat die Formel 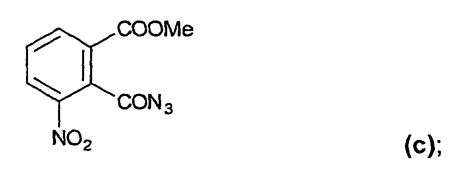
d) 28 g Verbindung **(c)** aus Schritt c) und 112 ml *tert*-Butanol wurden in einem 250 ml Rundkolben langsam erhitzt und 2 h unter Rückfluss gekocht. Die Reaktionsmischung wurde im Vakuum eingeengt und mittels Säulenchromatographie (PE : AcOEt, 16 : 1 bis 4 : 1) gereinigt. Das so erhaltene Rohprodukt lässt sich in Methanol zu gelben Kristallen umkristallisieren und hat die Formel 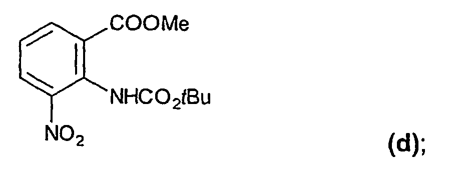
e) 10,8 g Verbindung **(d)** aus Schritt d) (36 mmol) und 4-Brombenzylbromid (40 mmol, 10,0 g) wurden in CH₃CN (200 ml) in einem 500 ml Rundkolben gelöst. Es wurde K₂CO₃-Pulver (36 mmol, 5,0 g) zugegeben. Die Reaktionsmischung wurde 10 h unter Rückfluss gekocht, im Vakuum eingeengt und mit Ethylacetat (2 x 500 ml) extrahiert. Die Extrakte wurden mit Wasser und einer wässrigen Lösung von NaCl gewaschen, über wasserfreiem Na₂SO₄ getrocknet, im Vakuum eingeengt und aus AcOEt/PE umkristallisiert, was die Zielverbindung **(e)** (15,4 g, Schmelzpunkt 107 - 108 °C) als farblose Kristalle ergab. Die Ausbeute betrug 92%. Analytische Daten der Zielverbindung **(e):** ¹H NMR (CDCl₃, 300 MHz): δ 1,30 (9H, s, t-Bu), 3,67 (3H, s, OMe), 4,57 (2H, dd, J=14,7 Hz, CH₂), 7,01 (2H, d, J=8,1 Hz, ArH), 7,32 (2H, d, J=8,1 Hz, ArH), 7,46 (1H, t, J=8,1 Hz, ArH), 7,89 (1 H, d, J=8,1 Hz, ArH), 8,00 (1H, d, J=8,1 Hz, ArH), ¹³C NMR (CDCl₃, 75 MHz): δ 27,8, 52,7, 53,1, 81,2, 121,9, 127,8, 128,1, 128,3, 131,0, 131,3, 131,6, 132,2, 134,8, 134,9, 135,3, 148,6, 153,5, 164,7; MS (EI) m/z (%): 464 (M⁺, 0,1), 365 (9), 348 (10), 316 (3), 302 (3), 235 (4), 185 (27), 169 (31), 57 (100). IR (film, cm⁻¹) λₘₐₓ = 3087, 2978, 2952, 1711, 1601, 1536, 1484, 1453, 1384, 1367, 1293, 1164, 1128, 1014, 984, 864, 766, 704.

### 2. Darstellung von

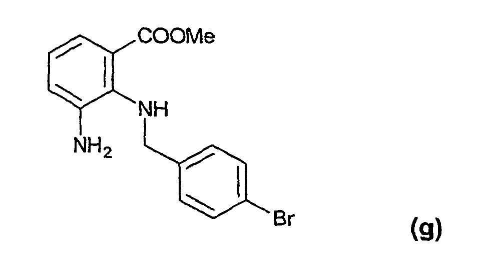

a) Verbindung **(e)** aus Beispiel 1 (3,2 mmol, 15,4 g) wurde in einer Mischung von CF₃COOH (32 ml) und CH₂Cl₂ (20 ml) in einem 100 ml Rundkolben gelöst und eine Stunde bei Raumtemperatur gerührt. Die Reaktionsmischung wurde in Vakuum eingeengt. Anschließend wurde Methanol (30 ml) hinzugefügt und der pH-Wert mit einer konzentrierten wässrigen NaHCO₃-Lösung auf etwa pH 10 eingestellt. Der gelbe Niederschlag wurde abfiltriert und aus Ethanol umkristallisiert, was die Verbindung der Formel 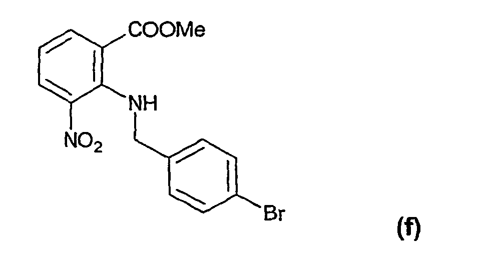 (9,45 g, Schmelzpunkt 111 -112 °C) in Form gelber, nadelförmiger Kristalle ergab. Die Ausbeute betrug 84%. Analytische Daten von (**f**): ¹H NMR (CDCl₃, 300 MHz) δ 3,87 (3H, s, OMe), 4,09 (2H, d, J=5,1 Hz, CH₂), 6,71 (1H, t, J=7,5 Hz, ArH), 7,16 (2H, d, J=8,4 Hz, ArH), 7,45 (2H, d, J=8,4 Hz, ArH), 7,96 (1H, d, J=7,5 Hz, ArH), 8,10 (1H, d, J=7,5 Hz, ArH), 8,77 (1H, br, ArH); ¹³C NMR (CDCl₃, 75 MHz) δ 50,2, 52,4, 115,0, 116,6, 121,8, 129,6, 131,6, 131,9, 136,6, 136,9, 137,4, 145,1, 167,7; MS (EI) m/z (%): 364 (M⁺, 2), 346 (16), 302 (15), 235 (13), 207 (8), 183 (100), 169 (80), 89 (64). IR (film, cm⁻¹) λₘₐₓ = 3306, 3094, 3011, 2953, 1937, 1715, 1692, 1601, 1575, 1527, 1486, 1441, 1402, 1339, 1260, 1198, 1116, 1073, 1010, 971, 893, 833, 808, 766, 734, 717, 670, 644, 589;
b) Verbindung **(f)** aus Schritt a) (7,4 g, 20 mmol) und trockenes Ethanol (40 ml) wurden in einem 100 ml Rundkolben vorgelegt. SnCl₂.2H₂O (102 mmol, 23,0 g) wurde portionsweise zugegeben. Die Reaktionsmischung wurde 2 h auf 80 °C erwärmt und das Ethanol entfernt. Der Rückstand wurde in Ethylacetat (60 ml) gelöst und der pH-Wert mit 4 N NaOH auf pH 11 - 12 eingestellt. Die organische Phase wurde abgetrennt und die wässrige Phase mit Ethylacetat extrahiert. Die vereinigten organischen Phasen wurden mit Wasser und einer wässrigen NaCI-Lösung gewaschen, über wasserfreiem Na₂SO₄ getrocknet und eingeengt. Das erhaltene Produkt, 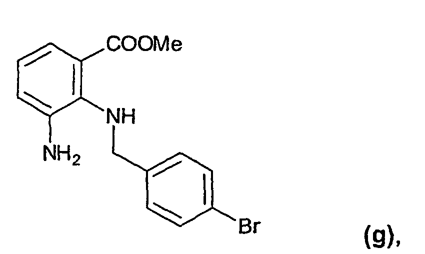 kann ohne weitere Reinigung direkt weiterverwendet werden. Analytische Daten von (**g**): ¹H NMR (DMSO, 300 MHz) δ 3,72 (3H, s), 4,36 (2H, s), 7,08 (1H, m), 7,31 (2H, brd, J=8,1 Hz), 7,44-7,51 (4H, m), 8.69 (3H, brs); ¹³C NMR (DMSO, 75 Hz) δ 51,1, 53,2, 121,9, 123,0, 124,4, 126,4, 127,2, 131,8, 131,9, 132,0, 132,1, 132,2, 136,2, 167,8; MS (ESI) [M+H]⁺ 335,0088 (berechnet 335,0089).

### 3. Darstellung von

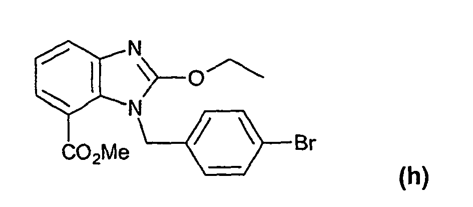

Verbindung (**g**) (31 mmol) aus Beispiel 2, C(OEt)₄ (46,5 mmol, 9,8 ml) und Essigsäure (31 mmol, 1,8 ml) wurden in einem 50 ml Rundkolben vermischt und 6 h bei 80 °C gerührt. Anschließend wurde die Reaktionsmischung eingeengt, der pH-Wert mit einer gesättigten NaHCO₃-Lösung auf pH 10 eingestellt, die Mischung mit Ethylacetat (2 x 500 ml) extrahiert, über wasserfreiem Na₂SO₄ getrocknet und unter vermindertem Druck eingeengt. Der erhaltene Feststoff wurde aus Ethylacetat umkristallisiert, was die Zielverbindung **(h)** (7,9 g, Schmelzpunkt 122 -123°C) ergab. Berechnet ausgehend von der eingesetzten Menge an Ausgangsverbindung **(f)** (s.o., Beispiel 2) betrug die Ausbeute 65%. Analytische Daten von **(h):** ¹H NMR (CDCl₃, 300 MHz) δ 1,45 (3H, t, J=7,2 Hz, Me), 3,74 (3H, s, OMe), 4,63 (2H, q, J=7,2 Hz, CH₂), 5,56 (2H, s, CH₂), 6,85 (2H, d, J=9 Hz), 7,16 (1H, t, J=8,4 Hz), 7,34 (2H, d, J=9 Hz), 7,57 (1H, d, J=8,4 Hz), 7,72 (1H, d, J=8,4 Hz); ¹³C NMR (CDCl₃, 75 MHz) δ 14,6, 46,7, 52,2, 66,7, 115,5, 120,9, 122,1, 123,7, 128,2, 131,5, 136,4, 141,9, 158,6, 166,7; MS (EI) m/z (%): 388 (M⁺, 14), 361 (2), 327 (3), 299 (2), 249 (5), 221 (5), 192 (3), 169 (100), 89 (28). IR (film, cm⁻¹) λₘₐₓ = 3407, 3058, 2991, 2952, 2852, 1903, 1709, 1615, 1549, 1479, 1430, 1382, 1248, 1128, 1036, 927, 869, 800, 743, 687.

### 4. Darstellung von

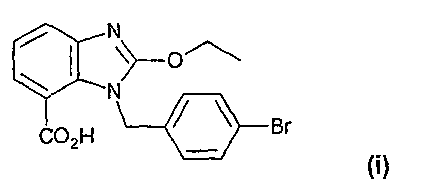

Verbindung **(h)** (10,3 mmol, 4,0 g), 1 M NaOH (30 ml) und Ethanol (30 ml) wurden in einem 100 ml Rundkolben vermischt und für 1 h bei 80°C gerührt. Anschließend wurde die Reaktionsmischung zur Entfernung des Lösungsmittels unter verminderten Druck eingeengt. Danach wurden Wasser (50 ml) und Ethylacetat (50 ml) zum Rückstand hinzugegeben und die wässrige Phase abgetrennt. Der pH-Wert wurde mit konzentrierter Salzsäure eingestellt auf pH 2 - 3, was Verbindung **(i)** als weißen Feststoff ergab, der im Vakuum getrocknet wurde; Ausbeute: 3,6 g, 95 %. Die so erhaltene Verbindung **(i)** kann ohne weitere Reinigung direkt weiterverwendet werden. Analytische Daten von (**i**): ¹H NMR (d-DMSO, 300 MHz): δ 1,36 (3H, t, J=6,9 Hz, CH₃), 4,56 (2H, q, J=6,9 Hz, CH₂), 5,55 (2H, s, CH₂), 6,89 (2H, d, J=8,1 Hz), 7,15 (1H, t, J=7,8 Hz), 7,43-7,51 Hz (3H, m), 7,64 (1H, d, J=8,1 Hz); ¹³C NMR (*d*-DMSO, 75 MHz): δ 15,0, 46,8, 67,2, 117,3, 120,9, 121,5, 122,2, 124,1, 129,2, 131,8, 132,1, 132,2, 137,7, 142,3,158,9, 168,1; MS (ESI) [M+H]⁺ 375,0193 (berechnet 375,0339).

### 5. Darstellung von

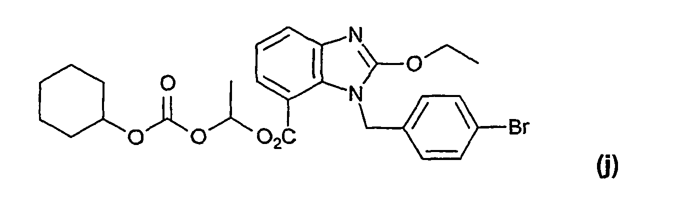

Verbindung **(i)** aus Beispiel 4 (9,7 mmol, 3,64 g), 1-{[(Cyclohexyloxy)carbonyl]-oxy}-1-iodethan **(k)** aus dem folgenden Beispiel 6 (19,5 mmol, 4,05 g), wasserfreies K₂CO₃ (9,7 mmol, 1,34 g), Nal (42,7 mmol, 6,4 g) und trockenes DMF (40 ml) wurden in einem 100 ml Rundkolben vermischt und 13 h bei 60 °C gerührt. Nachdem unter vermindertem Druck eingeengt wurde, wurde die Reaktionsmischung mit Ethylacetat (2 x 200 ml) extrahiert. Die organischen Phasen wurden abgetrennt und mit Wasser und einer wässrigen NaCI-Lösung gewaschen, über wasserfreiem Na₂SO₄ getrocknet, eingeengt und mittels Säulenchromatographie (PE : AcOEt, 16 : 1 bis 4 : 1) gereinigt, was die Zielverbindung **(j)** (2,8 g) ergab. Der pH-Wert der wässrigen Phase wurde mit konzentrierter Salzsäure auf pH 2 - 3 eingestellt. Die Reaktionsmischung wurde anschließend mit Ethylacetat (100 ml) extrahiert. Die organische Phase wurde abgetrennt, über wasserfreiem Na₂SO₄ getrocknet und eingeengt, um weitere 1,2 g der Verbindung **(j)** zu erhalten. Die Ausbeute betrug 79%. Analytische Daten von (**j**):¹H NMR (CDCl₃, 300 MHz) δ 0,84-1,94 (16H, m), 4,65 (3H, m, CH₂, CH), 5,24 (2H, s, CH₂), 6,88 (3H, m), 7,16 (1H, t, J=8,4 Hz), 7,34 (2H, d, J=8,1 Hz), 7,60 (1H, d, J=8,4 Hz), 7,73 (1H, d, J=8,4 Hz); ¹³C NMR (CDCl₃, 75 MHz) δ 14,6, 19,5, 23,6, 25,1, 31,3, 46,7, 66,7, 77,5, 91,6, 114,4, 120,9, 121,1, 122,7, 124,1, 128,6, 131,6, 131,9, 136,3, 141,9, 152,5, 158,6, 164,0; MS (FAB): gefunden 567,5 (M⁺+Na), 545,5 (M⁺+1); IR (film, cm⁻¹) λₘₐₓ = 3413, 2938, 2860, 1754, 1722, 1618, 1551, 1485, 1458, 1428, 1280, 1243, 1077, 1038, 1008, 989, 911, 871, 802, 747,608.

### 6. Darstellung von 1-{[(Cyclohexyloxy)carbonyl]oxy}-1-iodethan (k)

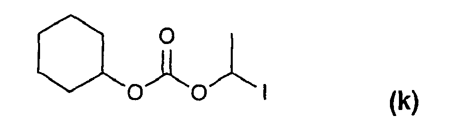

a) In einem 100 ml Rundkolben wurde bei -40 °C Triphosgen (10 mmol, 39,0 g) zu einer Suspension von Acetaldehyd (360 mmol, 20 ml) und PhCH₂N⁺Et₃Cl-(18 mmol, 4,1 g) gegeben. Die Reaktionsmischung wurde 5 h gerührt. Überschüssiges Triphosgen wurde bei vermindertem Druck entfernt. Der Rückstand wurde unter vermindertem Druck destilliert und das Destillat wurde gesammelt bei 41 - 42 °C/4,2 mm Hg, was 1-Chlorcarbonyloxy-1-chlorethan (Verbindung **(m)**) (21,2 g, 41,2% Ausbeute) ergab. Analytische Daten von (**m**): ¹H NMR (CDCl₃, 300 MHz) δ 1,85 (3H , d, J=5,7 Hz, CH₃), 6,42 (1H, q, J=5,7 Hz, CH).
b) In einem 250 ml Rundkolben wurde unter Eisbadkühlung Verbindung **(m)** aus Schritt a) (10 ml) tropfenweise zu einer Lösung von Cyclohexanol (91,5 mmol, 9,15 g) und Pyridin (91,8 mmol, 7,38 ml) in CH₂Cl₂ (150 ml) gegeben. Die Reaktionsmischung wurde 16 h bei Raumtemperatur gerührt, mit einer gesättigten, wässrigen NaCl-Lösung gewaschen, über wasserfreiem Na₂SO₄ getrocknet und anschließend das Lösungsmittel abdestilliert. Der Rückstand wurde unter vermindertem Druck destilliert und das Destillat bei 130-132°C/5 mm Hg gesammelt, was 1-{[(Cyclohexyloxy)carbonyl]oxy}-1-chlorethan (Verbindung **(n)**) (16,7 g) ergab. Analytische Daten von (**n**): ¹H NMR (CDCl₃, 300 MHz) δ 1,20-1,53 (6H, m, CH₂), 1,70 (2H, m, CH₂), 1,78 (3H, d, J=6 Hz, CH₃), 1,89 (2H, m, CH₂), 4,64 (1H, m, CH), 6,39 (1H, q, J=6 Hz, CH).
c) Verbindung **(n)** aus Schritt b) (6,7 mmol, 1,4 g) wurde in einem 100 ml Rundkolben in 50 ml MeCN gelöst. Nal (26,8 mmol, 4,4 g) wurde zugegeben und die Reaktionsmischung 90 min bei 60 °C gerührt. Nachdem unter vermindertem Druck eingeengt wurde, wurde der Rückstand mit Ether extrahiert. Die organische Phase wurde abgetrennt, über wasserfreiem Na₂SO₄ getrocknet und mittels Säulenchromatographie gereinigt, um die Zielverbindung **(k)** (810 mg, 40% Ausbeute) zu erhalten. Analytische Daten von **(k):** ¹H NMR (CDCl₃, 300 MHz) δ 1,23-1,93 (10H, m, CH₂), 2,23 (3H, d, J=6 Hz, CH₃), 4,68 (1H, m, CH), 6,75 (1H, q, J=6 Hz, CH).

### 7. Darstellung von Verbindung (o)

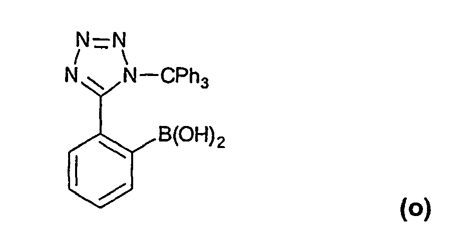

a) Benzonitril (10,3 g, 100 mmol), NH₄Cl (6,9 g, 1,3 eq), NaN₃ (8,5 g, 1,3 eq) und LiCI (300 mg) wurden in 100 ml DMF gelöst und die Reaktionsmischung wurde 12 h bei 100°C gerührt. Anschließend wurde der Großteil des Lösungsmittels unter vermindertem Druck entfernt. Der Rückstand wurde mit 10%iger wässriger NaOH bis zu einem pH-Wert von pH 12 alkalisch gemacht. Nach Extraktion mit Ethylacetat wurde die wässrige Phase abgetrennt und mit konzentrierter Salzsäure bis pH 2 angesäuert. Der Niederschlag wurde mit einem Büchner-Trichter abfiltriert, mit Wasser gewaschen und getrocknet, was Verbindung **(p)** 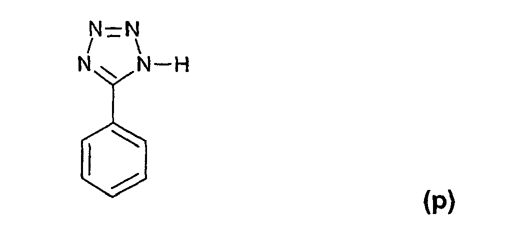 (13,5 g, Schmelzpunkt 208 - 209°C) ergab. Die Ausbeute betrug 96%. Analytische Daten von **(p):** ¹H NMR (d-DMSO, 300 MHz) δ 7,55-7,57 (3H, m), 8,01-8,03 (2H, m); ¹³C NMR (d-DMSO, 75 MHz) δ 129,5, 132,4, 134,8, 136,7, 160,7; MS (EI) m/z (%): 146 (M+, 42), 118 (100), 103 (17), 91 (46), 77 (32), 63 (48); IR (film, cm⁻¹) λₘₐₓ = 3055, 2982, 2837, 2607, 2545, 1607, 1562, 1485, 11463, 1409, 1163, 1056,1013, 725, 703, 686.
b) Verbindung **(p)** aus Schritt a) (6,6 g, 45 mmol) wurde in 20 ml CH₂Cl₂ gelöst und mit NEt₃ (8 ml, 1,3 eq) versetzt. Die Reaktionsmischung wurde in einem Eiswasserbad auf 0 °C abgekühlt und Ph₃CCl (13,2 g, 1,05 eq) wurde innerhalb von 10 min in 3 Portionen zugegeben. Anschließend wurde auf Raumtemperatur erwärmt und 3 h gerührt. Die Reaktionsmischung wurde filtriert, mit Wasser gewaschen und getrocknet um Verbindung **(q)** 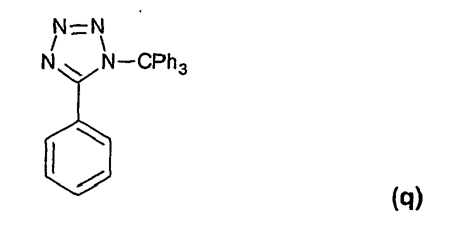 (16,5 g, Schmelzpunkt 163 -164 °C) zu erhalten. Die Ausbeute betrug 94%. Analytische Daten von **(q):** ¹H NMR (CDCl₃, 300 MHz) δ 7,21-7,24 (6H, m), 7,37-7,39 (9H, m), 7,47-7,49 (3H, m), 8,19-8,20 (2H, m); ¹³C NMR (CDCl₃, 75 MHz) δ 83,0, 127,0, 127,5, 127,7, 128,3, 128,7, 130,3, 141,3, 164,0; IR (film, cm⁻¹) λₘₐₓ = 3058, 1490, 1465, 1445, 1186, 1028, 874, 763, 748, 697, 635.
c) Eine Lösung von Verbindung **(q)** aus Schritt b) (10 g, 25,8 mmol) in THF (30 ml) wurde unter Argon (Schutzgasatmosphäre) auf -20 °C temperiert. Anschließend wurde BuLi (1 M, 27 ml, 1,05 eq) hinzugegeben. Die Temperatur wurde auf -5 °C erhöht und es wurde 1 h gerührt. In der Zwischenzeit fiel eine große Menge eines Feststoffs aus. Erneut wurde auf -25 °C abgekühlt und B(OMe)₃ (4,3 ml, 1,5 eq) über eine Spritze langsam hinzugegeben. Anschließend ließ man die Reaktionsmischung sich auf 20°C erwärmen und rührte sie eine halbe Stunde. Das Lösungsmittel wurde unter vermindertem Druck auf 1/3 der ursprünglichen Menge reduziert wobei sich weißer Feststoff bildete. Der Feststoff wurde abfiltriert, gewaschen mit 20% THF in H₂O (40 ml) und Wasser (40 ml) und getrocknet, was die Zielverbindung **(o)** (10,4 g) ergab. Die Ausbeute betrug 94%. Die Verbindung **(o)** kann ohne weitere Reinigung weiterverwendet werden.

### 8. Darstellung von Candesartan Cilexetil (C-C Kupplung)

Die Beispiele 8-a1) bis 8-a4) zeigen 4 mögliche Reaktionsbedingungen auf, wodurch eine C-C Kupplung der Verbindung **(j)** aus Beispiel 5 mit der Verbindung **(o)** aus Beispiel 7 erfolgen kann.

Beispiel 8-b) beschreibt das Entfernen der Schutzgruppe mit anschließender Aufarbeitung.
a1) Verbindung **(j)** aus Beispiel 5 (2,5 g, 4,6 mmol), Verbindung **(o)** aus Beispiel 7 (3,4 g, 1,2 eq) und Na₂CO₃ (1,46 g, 3 eq) wurden in 20 ml Toluol/Wasser (7 : 3) gelöst und das System dreimal mit Argon gespült. Anschließend wurde Pd(PPh₃)₄ (266 mg, 0,05 eq) hinzugegeben und die Reaktionsmischung 13 h auf 80 °C erhitzt. Die Reaktionsmischung wurde dann mit Ethylacetat extrahiert und mittels Säulenchromatographie (PE : Ether, 3 : 2) gereinigt um 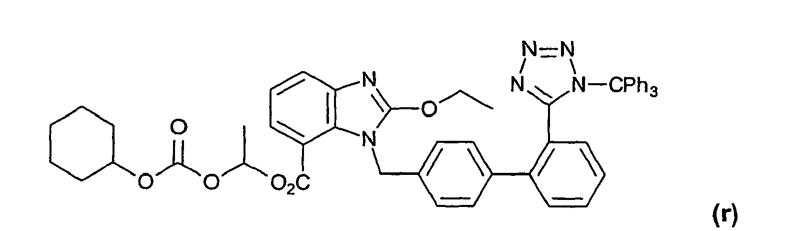 Verbindung **(r)** (3,2 g, 82% Ausbeute) zu erhalten. Analytische Daten von (**r**): ¹H NMR (CDCl₃, 400 MHz) δ 1,19-1,51 (11H, m), 1,67-1,71 (3H, m), 1,91 (2H, m), 4,59-4,65 (3H, m, CH₂ and CH), 5,56 (2H, q, J=16 Hz, CH₂), 6,78-7,47 (24H, m), 7,56 (1H, d, J=8 Hz), 7,76 (1H, d, J=8,4 Hz), 7,87 (1H, d, J=6,8 Hz); ¹³C NMR (CDCl₃, 100 MHz) δ 14,6, 19,5, 23,6, 25,1, 31,4, 47,0, 66,7, 77,5, 82,2, 91,7, 114,8, 120,8, 122,5, 124,0, 126,2, 126,3, 127,4, 127,6, 128,2, 129,4, 129,8, 130,2, 130,3, 130,6, 135,7, 140,0, 141,2, 141,8, 142,0, 152,5, 158,7, 163,9, 164,0; MS (FAB): gefunden 875 (M⁺ + Na), 853 (M⁺ + 1); IR (film, cm⁻¹) λₘₐₓ = 2939, 2860, 1753, 1723, 1550, 1447, 1429, 1279, 1242, 1078, 1036, 909, 733, 699.
a2) NiCl₂(PPh₃)₂ (33 mg, 0,05 mmol), PPh₃ (26 mg, 0,1 mmol) wurden unter Argon Schutzgasatmosphäre in 3 ml DME (Dimethoxyethan) oder Benzol gelöst. Anschließend wurde Butyllithium (0,13 ml, 0,2 mmol, 1,6 M in Hexan) zugetropft und es wurde für 10 min gerührt. Verbindung **(j)** aus Beispiel 5 (0,5 mmol), K₃PO₄ (1,5 mmol), Verbindung **(o)** aus Beispiel 7 (1,1 mmol) wurden hinzu gegeben und die Reaktionsmischung 12 h auf 80 °C erhitzt. Die Reaktionsmischung wurde zweimal mit Ethylacetat extrahiert und die organischen Phasen mit Wasser und gesättigter wässriger NaCI-Lösung gewaschen. Die organische Phase wurde abgetrennt, über wasserfreiem Na₂SO₄ getrocknet und mittels Säulenchromatographie gereinigt.
a3) Die Reaktion wurde wie in Beispiel 8-a2) beschrieben durchgeführt, wobei anstelle von Butyllithium alternativ DIBAH (Disobutylaluminiumhydrid) (0,045 ml, 0,2 mmol) verwendet wurde.
a4) NiCl₂(PPh₃)₂ (33 mg, 0,05 mmol), PPh₃ (26 mg, 0,1 mmol) und Zinkpulver (55 mg, 0,85 mmol) wurden unter Argon Schutzgasatmosphäre in 1 ml THF gelöst und es wurde für 1 h auf 50 °C erwärmt. Anschließend wurde Verbindung **(j)** aus Beispiel 5 (0,5 mmol), K₃PO₄ (1,5 mmol) und Verbindung **(o)** aus Beispiel 7 (1,5 mmol), sowie 2 ml THF hinzu gegeben. Die Reaktionsmischung wurde für 48 h zum Rückfluss erhitzt und wie unter a2) beschrieben aufgearbeitet.
b) Verbindung **(r)** aus Schritt a) (3 g, 3,5 mmol) wurde in 51 ml CH₂Cl₂ : MeOH : 1 N HCI (10 : 36 : 5,5) gelöst und die Reaktionsmischung 3,5 h bei Raumtemperatur gerührt. Anschließend wurde mit gesättigter, wässriger NaHCO₃ der pH-Wert etwa auf pH 3 eingestellt und der überwiegende Teil des Lösungsmittels unter vermindertem Druck entfernt. Der Rückstand wurde mit Ethylacetat extrahiert und mittels Säulenchromatographie (PE : AcOEt, 1 : 1) gereinigt um

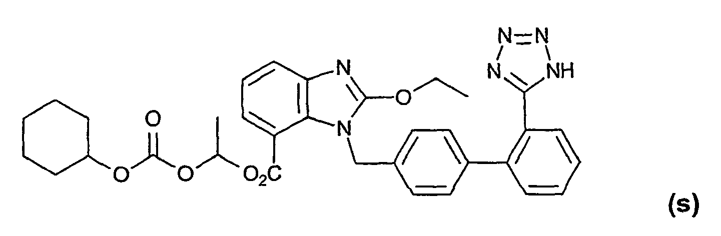

Candesartan Cilexetil **(s)** (2,05 g, Schmelzpunkt 128 - 129 °C) zu erhalten. Die Ausbeute betrug 95%. Analytische Daten von **(s):** ¹H NMR (CDCl₃, 300 MHz) δ 0,97-1,39 (11H, m), 1,46-1,48 (1H, m), 1,63 (2H, s, br), 1,78-1,82 (m, 2H), 3,92-4,00 (m, 1H), 4,28-4,36 (m, 1H), 4,46-4,52 (m, 1H), 5,55 (2H, dd, J=18, 20 Hz, CH₂), 6,55-6,60 (4H, m), 6,69-6,75(2H, m), 6,81 (1H, t, J=7,8 Hz), 7,24 (1H, d, J=7,8 Hz), 7,39 (1H, d, J=7,5 Hz), 7,52-7,61 (2H, m), 7,93 (1H, d, J=8,1 Hz); ¹³C NMR (CDCl₃, 75 MHz) δ 14,4, 19,0, 23,4, 24,9, 31,2, 46,7, 67,7, 77,6, 91,7, 115,3, 120,6, 121,2, 123,3, 124,1, 124,9, 128,2, 129,4, 130,2, 130,4, 131,1, 136,1, 138,0, 139,5, 140,8, 152,2, 154,7, 157,7, 163,1; MS (FAB): gefunden 633 (M⁺ + Na), 611 (M⁺ + 1); IR (film, cm⁻¹) λₘₐₓ = 3060, 2939, 2860, 1753, 1725, 1613, 1550, 1473, 1434, 1281, 1245, 1078, 1038, 992, 911, 730.

## Patentansprüche

1. Verfahren zur Herstellung von Candesartan, eines Candesartan-Salzes, oder eines Candesartan-Esters oder einer geschützten Form von Candesartan, eines Candesartan-Salzes, oder eines Candesartan-Esters, insbesondere Candesartan Cilexetil, das folgende Schritte umfasst:
(a) Bereitstellen und Umsetzen einer Verbindung der Formel (I) 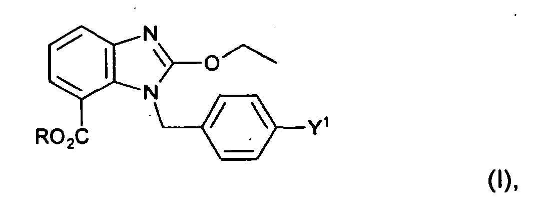 worin
- R Wasserstoff, ein unsubstituierter oder substituierter Alkyl- oder Arylrest, vorzugsweise Methyl oder (Cyclohexyloxycarbonyloxy)ethyl ist,
- Y¹ eine Gruppe ist, die unter Bildung einer C-C-Bindung in eine Kupplungsreaktion einzugehen vermag, in die ferner eine Gruppe Y² eingeht,
mit einer die Gruppe Y² aufweisenden Verbindung der Formel (II) 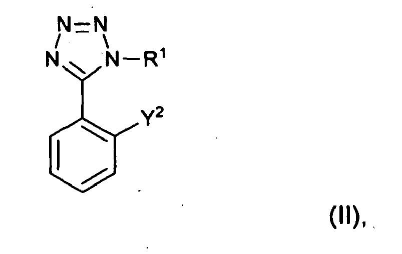
worin R¹ eine Tetrazolylschutzgruppe oder Wasserstoff ist, unter Bildung von Candesartan, einer geschützten Form von Candesartan oder eines Candesartan-Esters oder Candesartan Cilexetil,
wobei
Y¹ = B(OR⁴)₂, wobei jeder der Reste R⁴ unabhängig voneinander für Wasserstoff, Alkyl, Aryl oder Alkylaryl, bevorzugt für Wasserstoff steht, und Y² = Halogen, vorzugsweise Brom,
oder
Y¹ = Halogen, vorzugsweise Brom, und Y² = B(OR⁴)₂, wobei jeder der Reste R⁴ unabhängig voneinander für Wasserstoff, Alkyl, Aryl oder Alkylaryl, bevorzugt für Wasserstoff steht,
und gegebenenfalls
(b) Umwandeln in Candesartan, Candesartan Cilexetil oder in ein Salz.

2. Verfahren nach Anspruch 1, wobei R = Methyl ist.

3. Verfahren nach einem der Ansprüche 1 oder 2, wobei R ein Alkylrest, vorzugsweise Methyl, ist, und wobei Schritt (b) eine Umesterung des aus Schritt (a) resultierenden Esters umfasst.

4. Verfahren nach Anspruch 3, wobei Schritt (b) die Hydrolyse des aus Schritt (a) resultierenden Esters mittels Behandlung mit NaOH in EtOH umfasst.

5. Verfahren nach Anspruch 4, wobei Schritt (b) ferner das Umsetzen von Candesartan in Form der freien Carbonsäure mit einer Verbindung der Formel (IV) 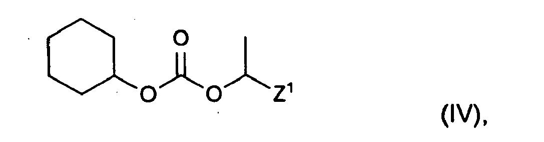 worin Z¹ eine Abgangsgruppe, vorzugsweise ein Halogen, vorzugsweise lod, ist,
unter Bildung von Candesartan Cilexetil,
vorzugsweise in Anwesenheit von Nal und K₂CO₃,
umfasst.

6. Verfahren nach einem der Ansprüche 1 bis 5, wobei R¹ aus einer der folgenden Gruppen ausgewählt ist: Wasserstoff, *tert*-Butyl und Triphenylmethyl, vorzugsweise Triphenylmethyl.

7. Verfahren nach einem der vorhergehenden Ansprüche, wobei ein oder mehrere Katalysatoren, vorzugsweise umfassend ein oder mehrere Übergangsmetalle, eingesetzt werden.

8. Verfahren nach Anspruch 7, wobei der Katalysator oder die Katalysatoren ausgewählt ist oder sind aus MnCl₂, CrCl₃, FeCl₂, Fe(acac)₃, FeCl₃, Fe(salen)Cl, CoCl₂(dppe), CoCl₂(dpph), Co(acac)₂, CoCl₂(dppb), Pd(PPh₃)₃ oder NiCl₂(PPh3)₂.

9. Verfahren nach Anspruch 7 oder 8, wobei der Katalysator zusammen mit einem Aktivator und/oder Stabilisator eingesetzt wird.

10. Verfahren nach Anspruch 8 oder 9, wobei der Katalysator oder die Katalysatoren ausgewählt sind aus der Gruppe der phosphanfreien, vorzugsweise Eisen enthaltenden Katalysatoren.

11. Verfahren nach einem der Ansprüche 7 bis 9, wobei eines oder mehrere der folgenden Lösungsmittel zum Einsatz kommt oder kommen: THF, THF/NMP, Et₂O, DME, Benzol, Toluol.

## Claims

1. Process for the preparation of candesartan, of a candesartan salt, or of a candesartan ester or of a protected form of candesartan, of a candesartan salt, or of a candesartan ester, in particular candesartan cilexetil, which comprises the following steps:
(a) preparation and reaction of a compound of the formula (I) 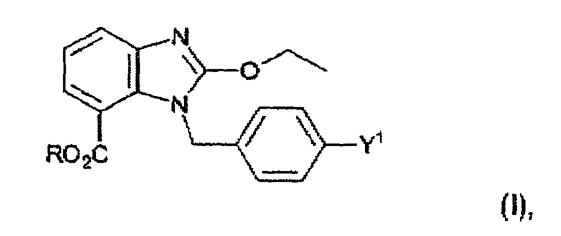 in which
- R is hydrogen, an unsubstituted or substituted alkyl or aryl radical, preferably methyl or (cyclo-hexyloxycarbonyloxy)ethyl,
- Y¹ is a group which is able to enter into a coupling reaction, into which a group Y² further enters, with formation of a C-C bond,
with a compound of the formula (II) containing the group Y² 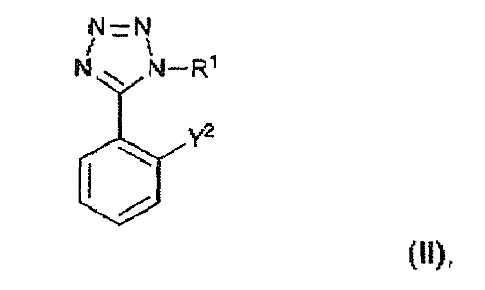
in which R¹ is a tetrazolyl protective group or hydrogen, with formation of candesartan, of a protected form of candesartan or of a candesartan ester or candesartan cilexetil,
wherein
Y¹ = B(OR⁴)₂, wherein each of the radicals R⁴ independently of one another represents hydrogen, alkyl, aryl or alkylaryl, preferably hydrogen, and Y² = halogen, preferably bromine, or
Y¹ = halogen, preferably bromine, and Y² = B(OR⁴)_{2,} wherein each of the radicals R⁴ independently of one another represents hydrogen, alkyl, aryl or alkylaryl, preferably hydrogen
and optionally
(b) conversion to candesartan, candesartan cilexetil or to a salt.

2. Process according to Claim 1, where R = methyl.

3. Process according to one of Claims 1 or 2, where R is an alkyl radical, preferably methyl, and where step (b) comprises a transesterification of the ester resulting from step (a).

4. Process according to Claim 3, where step (b) comprises the hydrolysis of the ester resulting from step (a) by means of treatment with NaOH in EtOH.

5. Process according to Claim 4, where step (b) furthermore comprises the reaction of candesartan in the form of the free carboxylic acid with a compound of the formula (IV) 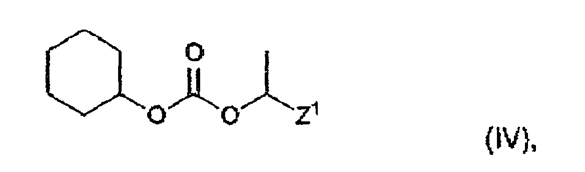 in which Z¹ is a leaving group, preferably a halogen, preferably iodine,
with formation of candesartan cilexetil,
preferably in the presence of Nal and K₂CO₃.

6. Process according to one of Claims 1 to 5, where R¹ is selected from one of the following groups: hydrogen, *tert*-butyl and triphenylmethyl, preferably triphenylmethyl.

7. Process according to one of the preceding claims, where one or more catalysts, preferably comprising one or more transition metals, are employed.

8. Process according to Claim 7, where the catalyst or the catalysts is or are selected from MnCl₂, Cryl₃, FeCl₂, Fe(acac)₃, Fecal₃, Fe(salen)CI, CoCl₂(dppe), CoCl₂(dpph), Co(acac)₂, CoCl₂(dppb), Pd(PPh₃)₃ or NiCl₂(PPh₃)₂.

9. Process according to Claim 7 or 8, where the catalyst is employed together with an activator and/or stabilizer.

10. Process according to Claim 8 or 9, where the catalyst or the catalysts is or are selected from the group consisting of the phosphane-free, preferably iron-containing catalysts.

11. Process according to one of Claims 7 to 9, where one or more of the following solvents is or are used: THF, THF/NMP, Et₂O, DME, benzene, toluene.

## Revendications

1. Procédé pour la préparation de candésartan, d'un sel de candésartan, ou d'un ester de candésartan ou d'une forme protégée de candésartan, d'un sel de candésartan, ou d'un ester de candésartan, en particulier de candésartan cilexétil, comprenant les étapes suivantes :
(a) préparation et réaction d'une composition ayant la formule (I) 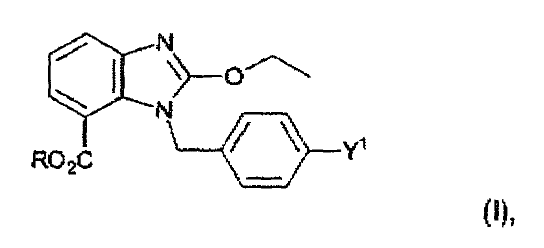 dans laquelle
- R signifie hydrogène, un résidu alkyl ou aryle, substitué ou non-substitué, préférablement méthyle ou (cyclohexyleoxycarbonyleoxy)éthyle
- Y¹ est un groupe capable de participer à une réaction de couplage à laquelle participe aussi un groupe Y² sous formation d'une liaison C-C, avec une composition comprenant le groupe Y² ayant la formule (II) 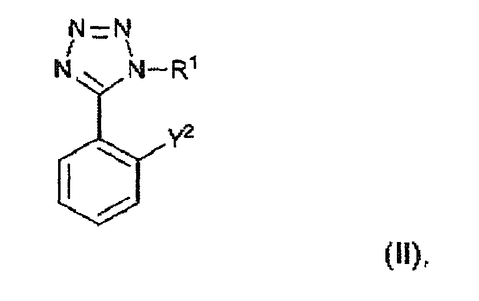
dans laquelle R¹ signifie un groupe protecteur du tétrazole ou hydrogène, sous formation de candésartan, d'une forme protégée de candésartan, ou d'un ester de candésartan ou de candésartan cilexétil,
Y¹ signifiant B(OR⁴)₂, chacun d'entre les résidus R⁴ désignant indépendamment l'un de l'autre hydrogène, alkyl, aryle ou alkylaryle, préférablement hydrogène, et Y² signifiant halogène, préférablement brome,
ou
Y¹ signifiant halogène, préférablement brome, et Y² signifiant B(OR⁴)₂, chacun d'entre les résidus R⁴ désignant indépendamment l'un de l'autre hydrogène, alkyl, aryle ou alkyl aryle, préférablement hydrogène,
et, le cas échéant,
(b) transformation en candésartan, candésartan cilexétil, ou en un sél.

2. Procédé selon la revendication 1, dans lequel R = méthyle.

3. Procédé selon l'une quelconque des revendications 1 ou 2, dans lequel R signifie un résidu alkyle, préférablement méthyle, et dans lequel l'étape (b) comprend une transestérification de l'ester résultant de l'étape (a).

4. Procédé selon la revendication 3, dans lequel l'étape (b) comprend l'hydrolyse de l'ester résultant de l'étape (a) par traitement avec NaOH en éthanol.

5. Procédé selon la revendication 4, dans lequel l'étape (b) comprend en plus la réaction du candésartan en forme d'acide carboxylique libre avec une composition ayant la formule IV 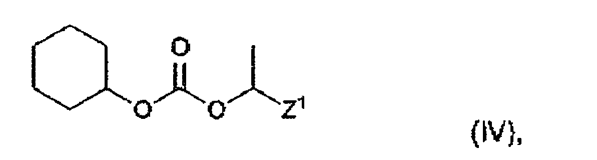 dans laquelle Z¹ signifie un groupe partant, préférablement un halogène, préférablement iode,
sous formation de candésartan cilexétil, préférablement en présence de Nal et de K₂CO₃.

6. Procédé selon l'une quelconque des revendications 1 a 5, dans lequel R¹ est choisi parmi l'un des résidus suivants: hydrogène, butyle tertiaire et triphényleméthyle, préférablement triphényleméthyle.

7. Procédé selon l'une quelconque des revendications précédentes, dans lequel un ou plusieurs catalyseurs préférablement comprenant un ou plusieurs métaux de transition sont employés.

8. Procédé selon la revendication 7, dans lequel le catalyseur ou les catalyseurs est ou sont choisi de parmi MnCl₂, CrCl₃, FeCl₂, Fe(acac)₃, FeCl₃, Fe(salen)CI, CoCl₂(dppe), CoCl₂(dpph), Co(acac)₂, CoCl₂(dppb), Pd(PPh₃)₃ ou NiCl₂(PPh₃)₂.

9. Procédé selon l'une quelconque des revendications 7 ou 8, dans lequel le catalyseur est employé ensemble avec un activateur et/ou un stabilisateur.

10. Procédé selon l'une quelconque des revendications 8 ou 9, dans lequel le catalyseur ou les catalyseurs sont choisi de parmi le groupe des catalyseurs sans phosphine préférablement contenant du fer.

11. Procédé selon l'une quelconque des revendications 7 a 9, dans lequel un ou plusieurs des solvants suivants est ou sont employés : THF, THF/NMP, Et₂O, DME, benzène, toluène.
